# EUROPEAN PATENT APPLICATION

(11) **EP 4 744 633 A1**
(43) Date of publication of application: **20.05.2026**
(21) Application number: 25183654.0
(22) Date of filing: 18.06.2025
(51) Int. Cl.: A61F 13/15, D04H 1/425

(54) **METHOD AND APPARATUS FOR MANUFACTURING A COMPONENT OF AN ABSORBENT PRODUCT FOR SANITARY USE**

(30) Priority: 18.11.2024 IT 202400025935
(71) Applicant: GDM S.P.A., 40133 Bologna (IT)
(72) Inventor: ZAVALLONI, Alessandro, 40133 Bologna (IT); CARUANA, Paolo, 40133 Bologna (IT)
(74) Representative: Emmi, Mario

(57) **Abstract**

The present invention concerns a method and an apparatus for manufacturing a component of an absorbent product for sanitary use.

Said method comprises the following steps:
- Moving forward, along a conveying path (P), an underlayer (22);
- Depositing, while said underlayer (22) is moved forward along said conveying path (P), an amount of natural fibres (30) on a surface of said underlayer (22) until forming a mass of said fibres (30);
- Depositing on said fibres (30), along said conveying path (P), at least one wetting agent (4) until obtaining a humidification percentage of said fibres comprised in the range 5% and 30% of relative humidity on the basis of the amount of dry fibres;
- Pressing, after said deposition step of said at least one wetting agent (4), said mass of fibres (30).

## Description

### Scope of the invention

The present invention relates to a method for manufacturing a component of an absorbent product for sanitary use.

Preferably, by absorbent product for sanitary use is meant any absorbent product for the hygiene of the user; purely by way of non-limiting example, said sanitary product can be represented for example by diapers, pads for adults, absorbent products for feminine hygiene, bed crosspieces, crosspieces for animal beddings.

Preferably, with component is meant any component configured to be assembled or integrated into any absorbent product for sanitary use; purely by way of non-limiting example, said component can be represented for example by at least one absorbent core (otherwise called absorbent "core").

The present invention also relates to an apparatus for manufacturing a component of an absorbent product for sanitary use.

Preferably, by absorbent product for sanitary use is meant any absorbent product for the hygiene of the user; purely by way of non-limiting example, said sanitary product can be represented for example by diapers, pads for adults, absorbent products for feminine hygiene, bed crosspieces, crosspieces for animal beddings.

Preferably, with component is meant any component configured to be assembled or integrated into any absorbent product for sanitary use; purely by way of non-limiting example, said component can be represented for example by at least one absorbent core (otherwise called absorbent "core").

The present invention finds advantageous application in the manufacture of components for absorbent products for sanitary use, in which it is necessary to guarantee a product with high absorption capacity, an optimal production flexibility, understood as the ability to manufacture different absorbent products for sanitary use, and a high productivity (understood as the number of absorbent products for sanitary use manufactured in a defined unit of time).

### A brief outline of the prior art

As is known, in recent years the technological development in the sector of the absorbent products for sanitary use has been oriented towards the development of devices and/or methods that would guarantee the manufacture of more absorbent products or that would guarantee a high productivity or that would guarantee an optimal production flexibility.

The use of the so-called "airlaid" material in the production of various products has long been known, including the absorbent products for sanitary use. This material is in fact used to create the pad, therefore the absorbent core, of the final absorbent product for sanitary use.

The "airlaid" material is a well-known material free of plastics and generally composed of cellulose fibres that are treated with air. SAP material (superabsorbent polymer) can be integrated if necessary.

This airlaid material is therefore characterised by being in the form of a layer of very uniform fibres, which is compact and produced with plants developed specifically to deposit these uniform layers of fibres and any SAP.

At the current state of the art, in the production of absorbent products for sanitary use, the generally known processes use mother reels of airlaid tape and with the entire tape suitable for constituting the absorbent pad or core simply by shaping it to size. The airlaid mother reel is therefore loaded into the machine and unrolled so that it is cut to size for the formation of the pad (herein referred to as the absorbent core). The pad, cut to size, is inserted between two layers of material in order to obtain the single finished absorbent products for sanitary use ready to be packaged and marketed.

In particular, at the current state of the art, the "airlaid" production lines are external to the converter (i.e. the production line) that produces the final absorbent product for sanitary use.

These "airlaid" reels, which will then constitute, as mentioned, the absorbent core of the finished product, are therefore produced on specific lines and with these reels that are often very wide (e.g. 2-3 m). Said reels are purchased in various formats and subsequently slid, and on order based on the necessary widths, in the converter.

The known technical solutions have several drawbacks.

A first drawback is represented by the low production flexibility that characterises the known technical solutions.

In particular, changing the width, grammage, presence or absence and quantity of SAP for the absorbent core, based on the type and/or size of absorbent product for sanitary use that is intended to be manufactured, requires those who produce the "raw material", or the airlaid reels, to vary these specifications upstream, and to have in stock all the purchased material formats (reels) which in themselves already make up a continuous tape of airlaid absorbent cores possibly provided with SAP.

A second drawback is represented by the non-optimal productivity, since in order to increase the absorption capacity of the absorbent core methods are used that require a technical time for their manufacture (for example, heating) and/or the deposition of chemical products that require a drying time non-conforming with high productivity of the absorbent core and therefore of the final product.

Finally, a third drawback is represented by the fact that some chemicals widely used in the production environment only marginally increase the absorbent capacity of the airlaid fibres, resulting in a poor absorption capacity or in any case a non-high absorption capacity of the absorbent core and therefore of the absorbent product for sanitary use comprising said absorbent core.

### Summary of the invention

Aim of the present invention is to provide a method for manufacturing a component of an absorbent product for sanitary use, which ensures that a product with high absorption capacity, an optimal production flexibility, understood as the ability to manufacture different absorbent products for sanitary use, and a high productivity (understood as the number of absorbent products for sanitary use manufactured in a defined unit of time) is obtained.

Furthermore, aim of the present invention is to provide an apparatus for manufacturing a component of an absorbent product for sanitary use, which ensures that a product with high absorption capacity, an optimal production flexibility, understood as the ability to manufacture different absorbent products for sanitary use, and a high productivity (understood as the number of absorbent products for sanitary use manufactured in a defined unit of time) is obtained.

According to the present invention, a method for manufacturing a component of an absorbent product for sanitary use, as defined in claim 1, is realized.

In particular, said method comprises a step of moving forward, along a conveying path (P), an underlayer (22).

Preferably, said method comprises a deposition step, while said underlayer (22) is moved forward along said conveying path (P), of an amount of natural fibres (30) on a surface of said underlayer (22) until forming a mass of said fibres (30).

Preferably, said method comprises a deposition step on said fibres (30), along said conveying path (P), of at least one wetting agent (4) until obtaining a humidification percentage of said fibres comprised in the range 5% and 30% of relative humidity (calculated on the basis of the amount of dry fibres).

Preferably, said method comprises a pressing step, after said deposition step of said at least one wetting agent (4), of said mass of fibres (30).

This solution solves all the aforementioned technical problems.

These characteristics derive from the experimental tests carried out by the Applicant, in which the Applicant tried different alternative solutions, to identify the solution that most guaranteed that a product with high absorption capacity, an optimal production flexibility, understood as the ability to manufacture different absorbent products for sanitary use, and a high productivity (understood as the number of absorbent products for sanitary use manufactured in a defined unit of time) was obtained.

In particular, the deposition of said at least one wetting agent 4, according to the range identified by the Applicant following experimental tests to identify the optimal humidification percentages, at said fibres 30 and/or said mass of fibres 30 involves the generation of hydrogen bridge chemical bonds between the fibres themselves.

This mode of generation of the hydrogen bridge chemical bonds overcomes in productivity the generation of the hydrogen bridges by heating, as they are generated between said fibres 30 according to lower timings according to the experimental tests conducted by the Applicant.

In addition, the hydrogen bridge chemical bonds thus generated increase the absorption capacity of the absorbent core, as they increase the compactness of the fibres.

In addition, the method thus configured allows to obtain a high production flexibility, as it is possible to vary in real time the amount of fibres 30 deposited and/or of said at least one wetting agent 4 on the basis of the component and/or the type of absorbent product for sanitary use to be manufactured.

According to a further aspect of the invention, an apparatus for manufacturing a component of an absorbent product for sanitary use, as defined in claim 13, is realized.

In particular, said apparatus comprises a conveying device ( 24, 25, 26) configured to convey, along a conveying path (P), an underlayer (22).

Preferably, said apparatus comprises a depositing device (10, 21 ) configured to deposit, while said underlayer (22) is moved forward along said conveying path (P), an amount of natural fibres (30) on a surface of said underlayer (22) until forming a mass of said fibres (30).

Preferably, said apparatus comprises at least one supplying station (10') configured to deposit on said fibres (30), along said conveying path (P), at least one wetting agent (4) until obtaining a humidification percentage of said fibres comprised in the range 5% and 30% of relative humidity (calculated on the basis of the amount of dry fibres).

Preferably, said apparatus comprises at least one pressing device (31) configured to press, downstream of said at least one supplying station (10'), said mass of fibres (30).

This solution solves all the aforementioned technical problems.

These characteristics derive from the experimental tests carried out by the Applicant, in which the Applicant tried different alternative solutions, to identify the solution that most guaranteed that a product with high absorption capacity, an optimal production flexibility, understood as the ability to manufacture different absorbent products for sanitary use, and a high productivity (understood as the number of absorbent products for sanitary use manufactured in a defined unit of time) was obtained.

In particular, the deposition of said at least one wetting agent 4, according to the range identified by the Applicant following experimental tests to identify the optimal humidification percentages, at said fibres 30 and/or said mass of fibres 30 involves the generation of hydrogen bridge chemical bonds between the fibres themselves.

This mode of generation of the hydrogen bridge chemical bonds overcomes in productivity the generation of the hydrogen bridges by heating, as they are generated between said fibres 30 according to lower timings according to the experimental tests conducted by the Applicant.

In addition, the hydrogen bridge chemical bonds thus generated increase the absorption capacity of the absorbent core, as they increase the compactness of the fibres.

In addition, the apparatus thus configured allows to obtain a high production flexibility, as it is possible to vary in real time the amount of fibres 30 deposited and/or of said at least one wetting agent 4 on the basis of the component and/or the type of absorbent product for sanitary use to be manufactured.

### Brief description of the drawings

For a better understanding of the present invention, a preferred embodiment is now described, by way of non-limiting example only, with reference to the attached drawing, in which:

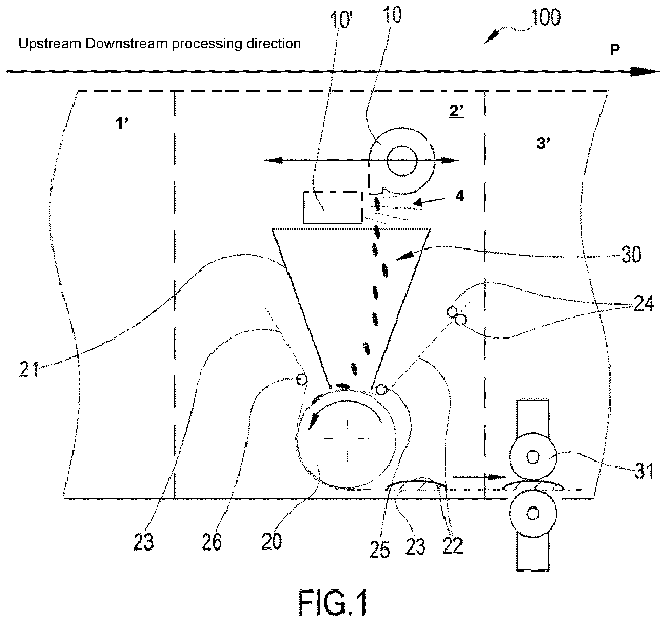

Figure 1 shows a view of a method and an apparatus for manufacturing a component of an absorbent product for sanitary use, according to an embodiment of the invention.

### Description of some preferred embodiments

Referring to Figure 1, the method of the present invention allows manufacturing a component of an absorbent product for sanitary use.

Preferably, by absorbent product for sanitary use is meant any absorbent product for the hygiene of the user; purely by way of non-limiting example, said sanitary product can be represented for example by diapers, pads for adults, absorbent products for feminine hygiene, bed crosspieces, crosspieces for animal beddings.

Preferably, with component is meant any component configured to be assembled or integrated into any absorbent product for sanitary use; purely by way of non-limiting example, said component can be represented for example by at least one absorbent core (otherwise called absorbent "core").

In particular, said method comprises a step of moving forward, along a conveying path (P), an underlayer 22.

Preferably, said method comprises a deposition step, while said underlayer 22 is moved forward along said conveying path (P), of an amount of natural fibres 30 on a surface of said underlayer 22 until forming a mass of said fibres 30.

Preferably, said method comprises a deposition step on said fibres 30, along said conveying path (P), of at least one wetting agent 4 until obtaining a humidification percentage of said fibres comprised in the range 5% and 30% of relative humidity (calculated on the basis of the amount of dry fibres).

Preferably, said method comprises a pressing step, after said deposition step of said at least one wetting agent 4, of said mass of fibres 30.

This solution solves all the aforementioned technical problems.

These characteristics derive from the experimental tests carried out by the Applicant, in which the Applicant tried different alternative solutions, to identify the solution that most guaranteed that a product with high absorption capacity, an optimal production flexibility, understood as the ability to manufacture different absorbent products for sanitary use, and a high productivity (understood as the number of absorbent products for sanitary use manufactured in a defined unit of time) was obtained.

In particular, the deposition of said at least one wetting agent 4, according to the range identified by the Applicant following experimental tests to identify the optimal humidification percentages, at said fibres 30 and/or said mass of fibres 30 involves the generation of hydrogen bridge chemical bonds between the fibres themselves.

This mode of generation of the hydrogen bridge chemical bonds overcomes in productivity the generation of the hydrogen bridges by heating, as they are generated between said fibres 30 according to lower timings according to the experimental tests conducted by the Applicant.

In addition, the hydrogen bridge chemical bonds thus generated increase the absorption capacity of the absorbent core, as they increase the compactness of the fibres.

In addition, the method thus configured allows to obtain a high production flexibility, as it is possible to vary in real time the amount of fibres 30 deposited and/or of said at least one wetting agent 4 on the basis of the component and/or the type of absorbent product for sanitary use to be manufactured.

Preferably, said deposition step of at least one wetting agent 4 comprises a conveying step of an air flow.

In other words, said at least one wetting agent 4 is deposited on said fibres 30 and/or on said mass of fibres 30 by conveying an air flow.

Preferably, said at least one wetting agent 4 comprises at least one substance selected from the group consisting of: water; alcohols, diols, triols and polyols; nitrogen compounds and relative oligomers and polymers; carboxylic acids; hydroxy acids; fluorinated compounds and relative oligomers and polymers; polyethers.

This group of substances was selected on the basis of the experimental tests carried out by the Applicant, in which the Applicant tried different alternative solutions, to identify the solution that most guaranteed a component (and therefore the relative product) with high absorption capacity, a high productivity and an optimal production flexibility.

In particular, for said group of substances used individually or in a composition comprising several substances falling within said group (and possibly added with further components such as stabilising agents and/or solvent agents), the Applicant has been able to verify through experimental tests that the generation of hydrogen bridge chemical bonds between the fibres 30 occurs in a completely effective manner, thanks to the presence of respective functional groups for each type of molecule, capable of generating a high number of hydrogen bridges with high intensity.

Furthermore, for said group of substances, the drying time of said at least one wetting agent 4 is strongly reduced, thus resulting in an optimal productivity of the process for manufacturing said component and the absorbent product for sanitary use comprising or incorporating said component.

According to a preferred embodiment, said fibres 30 comprise cellulose.

According to an even more preferred embodiment, said fibres 30 consist of cellulose.

Preferably, said deposition step of at least one wetting agent 4 comprises a step of nebulizing humid air.

In other words, said at least one wetting agent 4 is deposited on said fibres 30 and/or on said mass of fibres 30 by nebulizing humid air.

In this way, the conveying humid air can act as both conveying medium and wetting agent 4, both alone and in combination with at least one further wetting agent 4.

Preferably, said underlayer 22 comprises at least one belt and/or a tape and/or a conveyor wheel.

According to this embodiment, said fibres 30 and said at least one wetting agent 4 are deposited on said belt or tape or conveyor wheel.

Alternatively or additionally, said underlayer 22 comprises at least one carrier comprising cellulose and a thermoplastic polymer.

According to this embodiment, said fibres 30 and said at least one wetting agent 4 are deposited on said carrier, i.e. a tape comprising cellulose and any thermoplastic polymer. In this case, the hydrogen bridge chemical bonds are created only between the fibres 30 and the portion of cellulose present in the carrier.

According to a further preferred embodiment, said underlayer 22 comprises at least two carriers comprising cellulose and a thermoplastic polymer according to a sandwich-like conformation.

In detail, according to this embodiment, the two carriers (i.e., tapes) are arranged facing each other to form a sandwich-like conformation; the fibres 30, or the mass of fibres 30, and said at least one wetting agent 4 are deposited between the two carriers (remaining trapped in the sandwich-like conformation) and/or on the surface of one of the two carriers, in this way not remaining trapped in the sandwich-like conformation.

Furthermore, the hydrogen bridge chemical bonds are only created between the fibres 30 and the cellulose portion of at least one of the two carriers.

Preferably, said carrier is continuous.

According to a preferred embodiment, said fibres 30 are deposited on said at least one belt and/or tape and/or conveyor wheel and the carrier is conveyed so as to wrap around said fibres 30.

In other words, the carrier is wrapped around said fibres 30, with or without said at least one wetting agent 4 already deposited, which have been deposited on said at least one belt and/or tape and/or conveyor wheel.

Preferably, said method comprises a defibrating step of said fibres 30 from a starting material before depositing said fibres 30 on said underlayer 22.

Preferably, said method comprises a deposition step of an amount of at least one superabsorbent polymer on said mass of fibres 30 before the pressing step and before or after said deposition step of said at least one wetting agent 4.

According to this embodiment, the superabsorbent polymer, in combination with said fibres 30 or said mass of fibres 30, contributes to increasing the absorbent capacity of the component and the relative absorbent product for sanitary use.

Said at least one superabsorbent polymer may be deposited before the pressing step.

Furthermore, said at least one superabsorbent polymer may be deposited before or after said deposition step of the at least one wetting agent 4.

According to a further aspect of the present invention, referring to Figure 1, the apparatus 100 of the present invention allows manufacturing a component of an absorbent product for sanitary use.

Preferably, by absorbent product for sanitary use is meant any absorbent product for the hygiene of the user; purely by way of non-limiting example, said sanitary product can be represented for example by diapers, pads for adults, absorbent products for feminine hygiene, bed crosspieces, crosspieces for animal beddings.

Preferably, with component is meant any component configured to be assembled or integrated into any absorbent product for sanitary use; purely by way of non-limiting example, said component can be represented for example by at least one absorbent core (otherwise called absorbent "core").

In particular, said apparatus 100 comprises a conveying device (24, 25, 26) configured to convey, along a conveying path (P), an underlayer 22.

Preferably, said apparatus 100 comprises a depositing device (10, 21) configured to deposit, while said underlayer 22 is moved forward along said conveying path (P), an amount of natural fibres 30 on a surface of said underlayer 22 until forming a mass of said fibres 30.

Preferably, said apparatus comprises at least one supplying station 10' configured to deposit on said fibres 30, along said conveying path (P), at least one wetting agent 4 until obtaining a humidification percentage of said fibres comprised in the range 5% and 30% of relative humidity (calculated on the basis of the amount of dry fibres).

Preferably, said apparatus comprises at least one pressing device 31 configured to press, downstream of said at least one supplying station 10', said mass of fibres 30.

This solution solves all the aforementioned technical problems.

These characteristics derive from the experimental tests carried out by the Applicant, in which the Applicant tried different alternative solutions, to identify the solution that most guaranteed that a product with high absorption capacity, an optimal production flexibility, understood as the ability to manufacture different absorbent products for sanitary use, and a high productivity (understood as the number of absorbent products for sanitary use manufactured in a defined unit of time) was obtained.

In particular, the deposition of said at least one wetting agent 4, according to the range identified by the Applicant following experimental tests to identify the optimal humidification percentages, at said fibres 30 and/or said mass of fibres 30 involves the generation of hydrogen bridge chemical bonds between the fibres themselves.

This mode of generation of the hydrogen bridge chemical bonds overcomes in productivity the generation of the hydrogen bridges by heating, as they are generated between said fibres 30 according to lower timings according to the experimental tests conducted by the Applicant.

In addition, the hydrogen bridge chemical bonds thus generated increase the absorption capacity of the absorbent core, as they increase the compactness of the fibres.

In addition, the apparatus thus configured allows to obtain a high production flexibility, as it is possible to vary in real time the amount of fibres 30 deposited and/or of said at least one wetting agent 4 on the basis of the component and/or the type of absorbent product for sanitary use to be manufactured.

Preferably, said at least one supplying station 103 comprises a plurality of supplying stations, each being configured to deposit an amount of said at least one wetting agent 4.

According to this embodiment, each supplying station 103 is configured to supply a determined amount of a single wetting agent 4; this amount and/or wetting agent may be the same or different with respect to the amounts and/or wetting agents supplied by all or some of the other supplying stations 103.

Alternatively, each supplying station 103 is configured to supply a certain amount of a composition comprising several wetting agents 4; such amount and/or composition of wetting agents may be the same or different with respect to the amounts and/or compositions of wetting agents supplied by all or some of the other supplying stations 103.

Preferably, said at least one pressing device 31 comprises at least one pair of rollers 31for pressing.

Preferably, said rollers 31 have at least one smooth and/or patterned surface.

According to a preferred embodiment of the apparatus shown in Figure 1, the numbering 100 indicates a portion of the production line (i.e., the converter) for the production of a component of absorbent products for sanitary use.

Sections are therefore highlighted in succession (1', 2', 3') some of which are already known in the field in order to obtain the finished product and therefore not described in detail here.

The processing direction is therefore from "Upstream" (i.e. origin of the converter line) towards "Downstream" therefore towards the outlet section of the finished product.

The section 1' can be, as clarified below, a section in which the starting tape(s) is/are unrolled for the in-line formation of an absorbent core which is then inserted precisely to form the final absorbent product.

For this purpose, the section 1' (not shown in the figure in detail) can provide standard unwinders which, however, now, in accordance with the invention, do not directly unwind a ready airlaid tape but they unwind simple starting tapes (for example in TNT or cellulose) for the formation of an "ad-hoc" core as a function of needs.

Followed then by the sections 2', 3' for the formation of the absorbent core produced directly in the converter line according to the specific recipe and then the line continuing with the use of the core, as per prior art, for the formation of the finished product.

Therefore, entering more into the descriptive detail, and according to a possible embodiment of the invention as reported in Figure 1, there is now, in accordance with the invention and on board the converter machine 100, a specific section (indicated by way of non-limiting example with numbering 2') which contains in its inside a mould 20 for the in-line formation of the absorbent cores, i.e. fibres 30 preferably of cellulose with any SAP (superabsorbent polymer).

The mould 20 is therefore integrated inside the converter line 100 and therefore, in this case and in accordance with the invention, it is no longer necessary to use airlaid mother reels constituting the core once cut, since this is prepared "on site" in a special section 2' as described herein.

So, actually, according to the new production cycle, the absorbent core is produced on site within the converter which then continues within the converter 100 to be used to form the final absorbent product according to a final part of the cycle already known.

What is mentioned above, and as will be clarified immediately below, gives great flexibility to the production line 100, allowing the formats and the types of product to be made to be easily modified, also with considerable material savings.

The mould 20, as shown in Figure 1, can be for example in the form of a rotatable wheel (i.e. a roller) 20 to which at least one underlayer (according to one of the possible embodiments according to the present invention, as per Figure 1, a tape) 22, 23 of suitable starting material, for example made of a paper nature such as preferably cellulose or the like or in materials such as non-woven fabric, is conveyed.

Said at least one underlayer 22, 23 is conveyed, as described, by means of said conveying device along said conveying path (P).

Figure 1 therefore schematizes in the section 2' a sort of funnel or channel 21 that serves to convey the material, i.e. the cellulose fibres 30 that will form, in the production process, the absorbent cores.

The material that is poured into the mould 20 is therefore in the form of cellulose fibres 30.

As schematized in Figure 1, the fibres 30 can be poured into the mould by means of said depositing device 10, which can comprise any distributor/shredder configured to shred the cellulose to form the fibres 30, pouring the shredded product, for example by gravity or, better, by ejecting it through a pressure at the exit of a nozzle. Figure 1 therefore schematizes a depositing device 10 comprising a distributor/shredder device and a nozzle, through which said cellulose fibres 30 are conveyed into the mould 20.

The funnel or duct 21 typically helps precisely convey the fibres 30 on the mould.

The distributor / shredder device can also integrate said at least one supplying station 10' capable of sending a flow of said at least one wetting agent 4 to the cellulose material to humidify it, generating said hydrogen bridge chemical bonds.

The humidification operation can occur before the cellulose is shredded into fibres or as soon as it is shredded but preferably before it reaches the mould 20. The supplying station 10' can therefore be integrated into the distributor / shredder or it can be arranged separately, for example in such a way as to intercept the cellulose fibres 30 at the exit of the distributor / shredder device.

This allows, in the subsequent pressing step, to compact the whole much better thanks to the presence of said at least one wetting agent 4 in the cellulose fibres 30.

The mould is preferably obtained in a rotatable wheel 20 and more precisely a cylindrically shaped roller provided with a side surface (L). On its side surface, niches (N) can be obtained.

These niches (N) form the collection site for the fibres 30 that fall by gravity, as schematized in Figure 1, thus conforming each niche in a mould.

The mould has a geometry such that it replicates the shape and size of the final absorbent core as much as possible.

When the roller 20 rotates, for example in steps, it can stop each niche (N) under the duct 21 in order to receive the fibres 30.

More in particular, a first underlayer (in the case illustrated in Figure 1, a tape) 22 fed by the section 1' and moving along said conveying path (P), for example obtained by means of guide rollers, is laid on the niche, as schematized in Figure 1, in such a way that it covers the niche that has the function of mould, being filled with falling fibres 30.

The tape 22 is laid perfectly in the niche, thus replicating its shape, for example because it is sucked in by a suction. The niche obtained in the body of the roller 20 is not shown in Figure 1 for simplicity of description.

A second underlayer (in the case depicted in Figure 1, a tape) 23 is fed towards the rotatable mould 20 by a second feeding path with respect to that of the first tape 22, in such a way as to overlap the first tape 22 that covers the niche containing the fibres 30.

In this way, one of the possible absorbent core configurations according to the present invention and according to what is already described above is obtained.

Said tapes 22 and 23 overlapped in adherence to the roller 20 move integrally with the roller 20. For example, this is because the roller is provided with suction holes to keep the tape adhering to it, so rotating the roller causes it to move forward towards the following sections. In this way, the two overlapped tapes at the niche and containing the fibres move towards the further processing sections.

The roller 20 is, as mentioned, provided with a plurality of niches (N) (not shown in Figure 1 for simplicity of description) such that cyclically, during its rotation, it carries under the funnel 21 a respective niche with the tape laid on it to receive the fibres 30 and then the second tape is applied on top of the first to close the fibres.

In other words, the second tape 23 overlaps the first tape 22 determining in output a product made by the two tapes 22 and 23 overlapped on each other and containing in their inside the fibres 30 in the zone of the tape that was in the niche. The roller 20 rotates in start and stop mode and when the roller rotates to transfer a next niche under the duct 21, in this step the pouring of cellulose fibres 30 is interrupted. In this way a component comprising absorbent cores spaced out from each other according to a certain pitch is obtained.

The whole is then conveyed towards a section 3' where said at least one pressing element 31, for example said at least one pair of rollers 31 or a vertically movable cylinder, crushes the component obtained so far, which is thus pressed to reach thicknesses as thin as possible.

In order to optimize the obtained core, the two tapes 22 and 23, before being coupled of course, can pass in contact with a glue distribution roller in such a way as to maximize their relative adhesion to optimize the stability of the structure of said exemplary configuration of absorbent core realizable according to the present invention.

Then a step follows of cutting to size (also called cutting out) always in line before sending the single absorbent core obtained to form the final absorbent product.

As already described above, said component can for example provide two layers of cellulose consisting of the two starting tapes 22 and 23 within which, in addition to the fibres 30, also SAP particulate material can optionally be trapped.

In particular, according to the present invention, it can be provided to pour cellulose fibres 30 into the mould to create a fibre bed 30 on which the SAP can then be deposited and then proceed with pouring other fibres over the SAP to make the second layer.

In this way, the SAP is enclosed between two layers of fibres 30, remaining trapped and this allows the use of SAP with very low particle size because, being trapped, it is not sucked in and does not come out, risking the creation of sharp tips.

In all of the above cases, the fibres 30 may be humidified and/or at least one of the two starting tapes may be provided with a layer of adhesive.

Obviously, as already mentioned, the mould is preferably of the start and stop type, i.e. it stops under the duct or funnel 21 to be filled and the filling can provide for the fall of fibres and/or SAP, for example according to the above, i.e. a first layer of fibres on which the SAP is poured and then followed by a further layer of fibres for covering.

As already mentioned above, in order to press the cellulose fibres 30 giving compactness and thinness to the product being formed inside the mould, it is possible, for example, to exploit the "hydrogen bonding" system, i.e. adhesion between the fibres 30 given by chemical bonds of hydrogen bridge that is generated between the fibres 30 in particular by subjecting the fibres 30 to said at least one wetting agent and possibly to high pressures.

In this sense, humidifying through the device 10' therefore creates a hydrogen bonding effect.

The term *hydrogen bonding* therefore indicates the hydrogen bridge that is created with the presence of said at least one wetting agent and possibly by pressing.

Figure 1 therefore schematizes with the numbering 31 said at least one pressing device.

Such a pressing device 31 may be in the form of a translatable piston which crushes the tape.

However, in a preferred solution and as shown in Figure 1, said pressing device 31 may provide said at least one pair of rollers 31 opposed to each other and counter-rotating. They are therefore arranged in front of each other at a certain adjustable distance from each other (a gap). The distance between them can be of the order of a few tenths of millimetres as well as something close to zero. The two counter-rotating rollers, by rotating, take at the inlet the tape and crush it between them, thus reducing the thickness of the absorbent core.

The use of said at least one wetting agent 4 and possibly of an adhesive favours maintaining the compressed thickness after the completion of this operation and after cutting out.

The method for manufacturing a component of an absorbent product for sanitary use and the apparatus for manufacturing a component of an absorbent product for sanitary use , as per description indicated above, allow the above-mentioned purposes to be achieved, such as ensuring that a product with high absorption capacity, an optimal production flexibility, understood as the ability to manufacture different absorbent products for sanitary use, and a high productivity (understood as the number of absorbent products for sanitary use manufactured in a defined unit of time) is obtained.

Finally, it is clear that modifications and variants can be made to the method for manufacturing a component of an absorbent product for sanitary use and to the apparatus for manufacturing a component of an absorbent product for sanitary use without departing from the protective scope of the present invention, as defined in the appended claims.

## Claims

1. Method for manufacturing a component of an absorbent product for sanitary use, said method comprising the following steps:
- Moving forward, along a conveying path (P), an underlayer (22);
- Depositing, while said underlayer (22) is moved forward along said conveying path (P), an amount of natural fibres (30) on a surface of said underlayer (22) until forming a mass of said fibres (30);
- Depositing on said fibres (30), along said conveying path (P), at least one wetting agent (4) until obtaining a humidification percentage of said fibres comprised in the range 5% and 30% of relative humidity on the basis of the amount of dry fibres;
- Pressing, after said deposition step of said at least one wetting agent (4), said mass of fibres (30).

2. Method according to claim 1, wherein said deposition step of at least one wetting agent (4) comprises a conveying step of an air flow.

3. Method according to one or more of the previous claims, wherein said at least one wetting agent (4) comprises at least one substance selected from the group consisting of: water; alcohols, diols, triols and polyols; nitrogen compounds and relative oligomers and polymers; carboxylic acids; hydroxy acids; fluorinated compounds and relative oligomers and polymers; polyethers.

4. Method according to one or more of the previous claims, wherein said fibres (30) comprise cellulose.

5. Method according to one or more of the previous claims, wherein said deposition step of at least one wetting agent (4) comprises a step of nebulizing humid air.

6. Method according to one or more of the previous claims, wherein said underlayer (22) comprises at least one belt and/or a tape and/or a conveyor wheel.

7. Method according to one or more of the previous claims, wherein said underlayer (22) comprises at least one carrier comprising cellulose and a thermoplastic polymer.

8. Method according to one or more of the previous claims, wherein said underlayer (22) comprises at least two carriers comprising cellulose and a thermoplastic polymer according to a sandwich-like conformation.

9. Method according to one or more of the previous claims, wherein said carrier is continuous.

10. Method according to one or more of claims 6 to 9, wherein said fibres (30) are deposited on said at least one belt and/or tape and/or conveyor wheel and the carrier is conveyed so as to wrap around said fibres (30).

11. Method according to one or more of the previous claims, wherein a defibrating step of said fibres (30) from a starting material is provided before depositing said fibres (30) on said underlayer (22).

12. Method according to one or more of the previous claims, wherein a deposition step of an amount of at least one superabsorbent polymer on said mass of fibres (30) is provided before the pressing step and before or after said deposition step of said at least one wetting agent (4).

13. Apparatus (100) for manufacturing a component of an absorbent product for sanitary use, said apparatus (100) comprising:
- A conveying device ( 24, 25, 26) configured to convey, along a conveying path (P), an underlayer (22);
- A depositing device (10, 21 ) configured to deposit, while said underlayer (22) is moved forward along said conveying path (P), an amount of natural fibres (30) on a surface of said underlayer (22) until forming a mass of said fibres (30);
- At least one supplying station ( 10') configured to deposit on said fibres (30), along said conveying path (P), at least one wetting agent (4) until obtaining a humidification percentage of said fibres comprised in the range 5% and 30% of relative humidity on the basis of the amount of dry fibres;
- At least one pressing device ( 31) configured to press, downstream of said at least one supplying station ( 10'), said mass of fibres (30).

14. Apparatus according to claim 13, wherein said at least one supplying station ( 10') comprises a plurality of supplying stations, each being configured to deposit an amount of said at least one wetting agent (4).

15. Apparatus according to claim 13 or 14, wherein said at least one pressing device ( 31) comprises at least one pair of rollers ( 31) for pressing, said rollers ( 31) having at least one smooth and/or patterned surface.
